# EUROPÄISCHE PATENTSCHRIFT

(11) **EP 0 702 998 B1**
(45) Veröffentlichungstag und Bekanntmachung des Hinweises auf die Patenterteilung: **17.09.1997**
(21) Anmeldenummer: 95111563.3
(22) Anmeldetag: 22.07.1995
(51) Int. Cl.: B01D 61/44, C07D 307/33, C07C 67/02, C07C 69/72

(54) **Verfahren zur Herstellung von Ketoverbindungen**
Process for preparing keto compounds
Procédé de préparation de composés cétoniques

(30) Priorität: 22.09.1994 DE 4433823
(43) Veröffentlichungstag der Anmeldung: 27.03.1996
(73) Patentinhaber: HÜLS AKTIENGESELLSCHAFT, 45764 Marl (DE)
(72) Erfinder: Sridhar, Srinivasan, Dr., D-45770 Marl (DE)

(56) Entgegenhaltungen:
- DE-A- 4 233 191
- PATENT ABSTRACTS OF JAPAN vol. 7 no. 201 (C-184) ,6.September 1983 & JP-A-58 099473 (DAINIPPON INK KK) 13.Juni 1983,

## Beschreibung

Die Erfindung betrifft ein neues Verfahren zur Herstellung von Ketoverbindungen aus den zugehörigen Alkalisalzen.

Ketoverbindungen sind wertvolle Zwischenverbindungen bei der Herstellung von zum Beispiel Heterocyclen, Pharmazeutika, Pflanzenschutzmitteln und Aromastoffen.

Durch Esterkondensation können Carbonsäureester zu Dicarbonylverbindungen umgesetzt werden. Nach dieser Methode kann Acetessigester aus Essigester hergestellt werden. Auch Lactone können unter Einsatz von Essigester in die entsprechenden Acetylderivate übergeführt werden. Wegen der relativ geringen Reaktivität der Estercarbonylgruppe werden als Kondensationsmittel starke Basen erforderlich. Im allgemeinen werden hierfür Alkalialkoholate verwendet. Diese Verbindungen können durch Umsetzung eines Alkohols mit dem Alkalimetall oder Laugen hergestellt werden. Die Herstellung der Alkoholate kann im elektrischen Feld, beispielsweise nach dem Amalgamverfahren oder gemäß DE-A-42 33 191 durch Elektrodialyse, erfolgen.

Die Esterkondensation von Lactonen mit Carbonsäureestern ist eine Methode zur Herstellung von Acyllactonen. Aufgrund der Empfindlichkeit mancher Lactone werden als Kondensationsmittel gemäß Angew. Chemie 71, 709 - 722, (1959), Natrium, Kalium, Na-Methylat, -Hydrid, -Amid sowie Diisopropylmagnesiumbromid und Triphenylmethylnatrium bevorzugt verwendet. Zur Herstellung von 2-Acetylbutyrolacton aus γ-Butyrolacton und Essigestern wurden Verfahren entwickelt, wobei Natrium, NaH und Na-Alkoholate (JP-B-45 009 538) als Kondensationsmittel eingesetzt werden. In einem weiteren Verfahren (JP-A-58 099 473) werden Lösemittel, wie Dimethylformamid, bei der Umsetzung von γ-Butyrolacton und Ethylacetat mit Natriummethylat zu 2-Acetylbutyrolacton benötigt. Auch Säurehalogenide werden als mögliche Kondensationsmittel erwähnt (JP-A-58 162 585)

Kennzeichnend für die Esterkondensation sind die folgenden Merkmale: Das Kondensationsmittel muß stets in mindestens äquimolaren Mengen (1 Mol je Mol β-Ketoverbindung) eingesetzt werden. Der entsprechende Verbrauch des Mittels wird unvermeidlich. Ferner stellt das zunächst entstehende Produkt ein Alkalisalz der β-Ketoverbindung dar (Patai, S., The Chemistry of the carbonyl group, London 1966, Seiten 273 - 276). Folglich hat man grundsätzlich ein Salz als Vorprodukt vorliegen, das anschließend in einem zweiten Schritt in die gewünschte freie β-Ketoverbindung übergeführt werden muß. Dies erfolgt dadurch, daß man dem Salz eine starke Säure zusetzt (GB-A-0 681 196). In diesem Schritt der Protonierung werden die freie β-Ketoverbindung und das Alkalisalz der zugeführten Säure gewonnen. Die einzelnen Reaktionsschritte werden am Beispiel von Acetessigester veranschaulicht, wobei das Salz des Ketoesters in der enolischen Form dargestellt ist:

### 1. Kondensation:

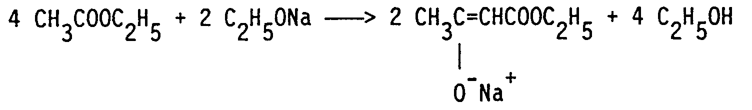

### 2. Protonierung:

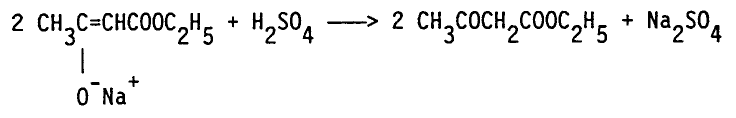

Die Einführung des Alkalimetallions durch das Kondensationsmittel führt zwangsläufig zum Anfall von Alkalisalz nach der Protonierung (Na₂SO₄ im obigen Beispiel). Da es sich hier nicht um katalytische, sondern um molare Mengen handelt, hat man bei den Umsetzungen mit einer relativ großen Zwangsproduktion vom Salz zu rechnen. Einerseits wird das wertvolle Kondensationsmittel verbraucht und andererseits fällt ein wertloses Salz an, das noch entsorgt werden muß.

Die Aufgabe der Erfindung besteht darin, eine Methode bereitzustellen, die die beiden obengenannten Nachteile überwindet.

Die Aufgabe wird erfindungsgemäß dadurch gelöst, daß man das Alkalisalz in einem nichtwäßrigen Medium ohne Zufuhr von systemfremder Säure elektrodialytisch protoniert und dabei die Ketoverbindung freisetzt.

Geeignete Alkalisalze sind vorzugsweise die Natrium- und Kaliumsalze.

Die Ketoverbindungen können einfache Ketone, Diketone oder Ketohalogenide, -aminosäuren oder -nitroverbindungen sein. Vorzugsweise handelt es sich um β-Ketoester und im besonderen um β-Ketolactone.

Die Elektrodialyse wird vorzugsweise in einer elektrodialytischen Zelle mit 2n + 2 Kammern, wobei n eine Zahl von 1 bis 50 ist, durchgeführt. In besonders bevorzugten Fällen ist n eine Zahl von 1 bis 10.

Geeignete nichtwäßrige Medien sind polare Lösemittel, wie z. B. Alkohole mit 1 bis 5 C-Atomen, wie Methanol, Ethanol, Isopropanol oder tert.-Butanol, Diole, wie Glykol und Butandiol, oder Triole, wie Glycerin. Verwendbar sind auch Chlorkohlenwasserstoffe, wie Methylenchlorid und Chloroform, Ether, wie Tetrahydrofuran, und Amide, wie Formamid und Dimethylformamid. Es können auch Lösemittelmischungen eingesetzt werden. Die Lösemittel können bis zu 5 % Wasser enthalten. Sie können auch ein Edukt oder ein Produkt enthalten.

Vorzugsweise besteht das Medium aus einem Alkohol, so daß man bei der Gewinnung der Ketoverbindungen als Nebenprodukt ein Alkalialkoholat herstellen kann, das für Esterkondensationen einsetzbar ist.

Während man gewöhnlich die Protonierung durch Einfuhr einer systemfremden Säure, wie Schwefelsäure, verwirklicht, wird bei der Erfindung auf die zwangsweise Einfuhr einer systemfremden Säure und eines systemfremden Anions, wie des Sulfat-Ions, verzichtet.

Die Elektrodialyse erfolgt vorzugsweise bei -20 bis +90 °C. Dabei werden Temperaturen von 10 bis 50 °C ganz besonders bevorzugt.

Das Verfahren hat den Vorteil, daß kein die Umwelt belastendes und auszuschleusendes Salz gebildet wird. Statt dessen kann ein Kondensationsmittel für die Esterkondensation zurückgewonnen werden.

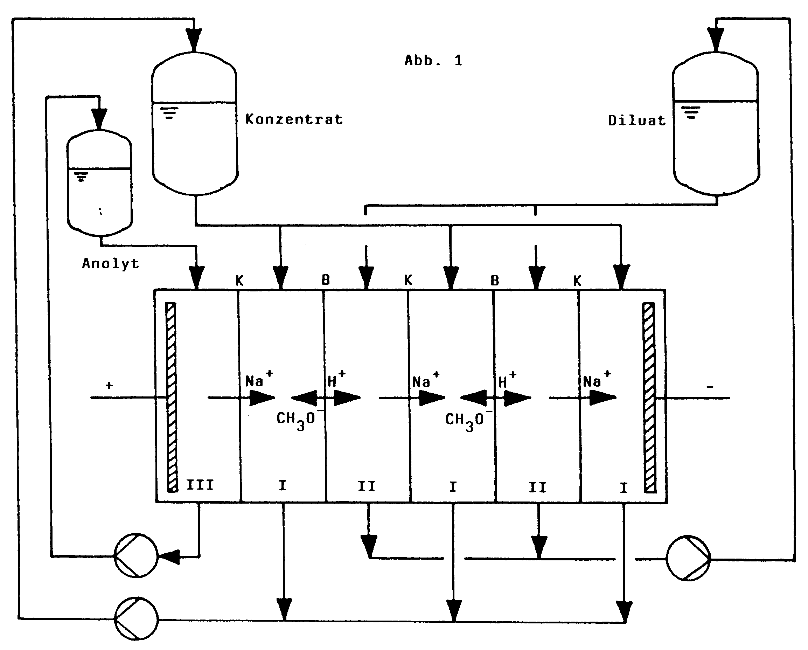

Die Vorgehensweise wird am Beispiel einer elektrodialytischen Zelle bestehend aus 6 Kammern gemäß Abbildung 1 erläutert: Die Kammern werden jeweils durch eine Kationenaustauschmembran K und eine bipolare Membran B begrenzt. Anstelle der bipolaren Membran kann auch ein Membranpaar, bestehend aus einer Anionen- und einer Kationenaustauschermembran, verwendet werden. Dabei wird vorzugsweise die Anionenaustauschermembran der Anode zugewandt und die Kationenaustauschermembran der Kathode zugewandt eingesetzt. Die Membranen können handelsübliche Membranen sein, sofern sie im gewählten System stabil sind.

In die Kammern I wird eine Lösung, die den Alkohol enthält, geleitet. Diese Lösung wird "Konzentrat" genannt. Die Kammern I werden der Anode zugewandt durch Kationenaustauschmembranen und der Kathode zugewandt durch bipolare Membranen begrenzt. Durch die Kathodenkammer, die durch eine Kationenaustauschmembran von der benachbarten Kammer getrennt ist, wird ebenfalls "Konzentrat" geleitet. Durch die Einwanderung von Alkaliionen aus den benachbarten Kammern II bzw. aus der Anodenkammer III entsteht hier das Alkalialkoholat.

Durch die benachbarten Kammern II, begrenzt durch bipolare Membranen (der Anode zugewandt) und Kationenaustauschmembranen (der Kathode zugewandt), wird eine alkoholische Salzlösung der Ketoverbindung gepumpt. Diese Lösung wird als "Diluat" bezeichnet. Beim Anlagen eines elektrischen Feldes wandern die Alkaliionen aus den Kammern II durch die Kationenaustauschmembranen K in Richtung der Kathode in die Kammern I und bilden dort das Alkoholat. Durch die bipolaren Membranen B treten Protonen. Deshalb erfolgt in den Kammern II die Protonierung des Salzes, wodurch die freie Ketoverbindung entsteht. Ein Kammerpaar, bestehend aus den Kammern I und II, stellt eine funktionelle Zelle dar. Der Zulauf beider Lösungen, des "Konzentrats" und des "Diluats", in die entsprechenden Kammern erfolgt parallel.

Die Kathodenkammer kann mit einer eigenen Alkoholatlösung versorgt werden. Man kann sie auch gemäß Abbildung 1 als eine "Konzentrat"-Kammer benutzen, da auch hier die Alkaliionen durch die Kationenaustauschmembran eintreten. In der Anodenkammer III kann als Anolyt eine alkoholische Lösung von Natriumacetat vorgelegt werden. Alternativ kann man auch hier das "Diluat" selbst als Kationenquelle einführen (nicht in Abbildung 1 ausgeführt), sofern keine unerwünschte Reaktionen an der Anode zu befürchten sind. Die Elektrodialyse kann absatzweise oder kontinuierlich ausgeführt werden. Weitere Einzelheiten zur praktischen Ausführung werden anhand der Beispiele verdeutlicht.

In einer besonderen Ausführung des erfindungsgemäßen Verfahrens gewinnt man einen β-Ketoester aus der Salzform und führt gleichzeitig eine Umesterung durch. Dieses ist dann möglich, wenn der Alkohol des "Konzentrats" von dem Alkohol des "Diluats" verschieden ist. So kann beispielsweise aus dem Na-Salz des Acetessigsäureethylester der freie Acetessigsäuremethylester gewonnen werden.

Das erfindungsgemäße Verfahren ist vielseitig verwendbar. Die Anwendung des Verfahrens erstreckt sich auf viele Reaktionen, bei denen ein Reagens verbraucht wird und bei denen durch die Erfindung eine Wiedergewinnung, eine Bereitstellung und eine Wiederverwendung dieses Reagens für die Reaktion ermöglicht wird. Außer Alkalialkoholaten können auch andere wertvolle Verbindungen wiedergewonnen werden.

Die Erfindung betrifft vorzugsweise die Claisen-Kondensation von einfachen Estern und die Dieckmann-Kondensation von Diestern, wobei cyclische Ketoester und nach Verseifung und Decarboxylierung cyclische Ketone, wie zum Beispiel Chromanon, Cumaranon, Cyclohexandion, Hydrindon, Piperidon, Thiopyranon oder Indoxylsäure (eine verkappte Ketocarbonsäure), entstehen. Die Dieckmann-Kondensation wird auch für Ringschlüsse bei der Steroidsynthese und bei cyclisierenden Umlagerungen von Heterocyclen angewandt. Das erfindungsgemäße Verfahren eignet sich auch für die durch Alkoholat katalysierte Umsetzung von Malonsäurediester mit Formamid zu 2,6-Dihydroxypyrimidin (ein verkapptes Keton).

Daneben kann das Verfahren auch auf andere Reaktionen zur Herstellung von Ketoverbindungen angewandt werden. So wird auch bei der Umacylierung von β-Ketoestern (Einführung einer neuen Acylgruppe über ein Säurehalogenid) eine alkoholische Alkoholatlösung als Reagens benötigt, das auf dem erfindungsgemäßen Wege zurückgewonnen werden kann.

Bei den unten angeführten Beispielen wird gemäß Abbildung 1 eine elektrodialytische Zelle mit 6 Kammern verwendet. Die Membranen weisen jeweils eine Fläche von 100 cm² auf. Die Lösungen werden aus den Vorratsbehältern in die Kammern gepumpt und wieder in die Behälter zurückgeführt. Das "Diluat" bildet einen Kreislauf. Das "Konzentrat" sowie das Katholyt bilden gemeinsam einen zweiten Kreislauf. Die Anode wird ähnlich mit einem weiteren Kreislauf versehen. Als Elektroden werden eine Anode aus platiniertem Titan und eine Kathode aus Stahl verwendet. Die folgenden Membranen werden eingesetzt:
- Kationenaustauschmembranen vom Typ C66-10F von Tokuyama Soda, Japan
- Bipolare Membranen vom Fraunhofer-Institut für Grenzflächen- und Bioverfahrenstechnik, Stuttgart

Für die Elektrodialyse wird eine Spannung von 40 V angelegt. Die Ströme in den Kreisläufen entsprechen jeweils 11 l/h.

### Zur Analyse

Die Ermittlung des im Konzentrat entstandenen Natriummethylats erfolgt potentiometrisch nach einer entsprechenden Eichung. Die Natrium-Bestimmung erfolgt durch eine Elementaranalyse. Nach jedem Versuch wird das Diluat in einem Rotationsverdampfer im leichten Vakuum von Methanol befreit und anschließend über einen Claisen-Aufsatz bei 1 hPa destillativ aufgearbeitet. Aus den gaschromatographischen Analysen der Fraktionen wird die in freier Form vorliegende Ketoverbindung ermittelt.

### Beispiel 1

Als Diluat werden 1 586 g einer 1,66 gew.-%igen Lösung von Na-Acetylbutyrolacton in Methanol vorgelegt. Das Konzentrat sowie das Katholyt bestehen anfänglich aus 1 695 g einer Lösung von 1,23 Gew.-% Natriummethylat in Methanol. In der Anodenkammer werden 1 654 g einer 12 gew.-%igen methanolischen Natriumacetatlösung vorgelegt. Die Elektrodialyse wird bei 23 °C durchgeführt und nach 22 Stunden abgebrochen.

Man erhält 11,65 g freies Acetylbutyrolacton im Diluatkreislauf und 50,62 g Natriummethylat im Konzentratkreislauf.

### Beispiel 2

Der Versuch verläuft über 23 Stunden gemäß Beispiel 1 mit den Ausgangsmengen von 1 686 g Diluat und 1 516 g Konzentrat, als Anolyt werden aber anstelle einer Lösung von Natriumacetat weitere 1 456 g Diluat eingesetzt.

Es entstehen 14,1 g freies Acetylbutyrolacton im Diluatkreislauf und 27,85 g Natriummethylat im Konzentratkreislauf.

### Beispiel 3

Mit den Ausgangsmengen von 1 688 g Diluat, 1 528 g Konzentrat und 1 428 g Anolyt wird wie in Beispiel 1 verfahren. Die Elektrodialyse wird jedoch bei 12 °C durchgeführt und erst nach 51 Stunden abgebrochen.

Es entstehen 22,65 g freies Acetylbutyrolacton im Diluatkreislauf und 68,41 g Natriummethylat im Konzentratkreislauf.

### Beispiel 4

Als Diluat werden 1 268 g einer 7 gew.-%igen Lösung von Na-Acetessigsäureethylester in Methanol vorgelegt. Das Konzentrat sowie das Katholyt bestehen anfänglich aus 1 260 g einer Lösung von 1,25 Gew.-% Natriummethylat in Methanol. In die Anodenkammer werden 1 400 g einer 12 gew.-%igen methanolischen Natriumacetatlösung vorgelegt. Die Elektrodialyse wird bei 22 °C durchgeführt und nach 28 Stunden abgebrochen.

Man erhält 37 g freien Acetessigsäuremethylester neben 1,5 g Acetessigsäureethylester im Diluatkreislauf und 62 g Natriummethylat im Konzentratkreislauf.

Demnach ist hier die Umesterung unter elektrodialytischen Bedingungen bereits nach 28 Stunden zu 97 % erfolgt.

### Vergleichsbeispiel A

Zur Umesterung unter neutralen Bedingungen: Zwei identische Gemische aus 47,2 g freiem Acetessigsäureethylester und 47,2 g Methanol werden 70 Stunden lang bei 22 °C in einem Kolben gerührt. Bei einem Gemisch werden jeweils 20 cm² der bei den obigen Versuchen verwendeten bipolaren Membran sowie der Kationenaustauschmembran eingetaucht. Am Ende entstehen jeweils 14 g Acetessigsäuremethylester. Das entspricht einem Umsatz von nur 30 %.

### Beispiel 6

Als Diluat werden 1 257 g einer 9,8 gew.-%igen Lösung von Na-Acetessigsäuremethylester in Methanol vorgelegt. Das Konzentrat wird vom Katholyt getrennt. Jede Lösung bildet einen eigenen Kreislauf. Das Konzentrat besteht anfänglich aus 1 228 g einer Lösung von 1,26 Gew.-% Natriummethylat in Methanol. Als Katholyt werden 1 211 g der gleichen Lösung getrennt vorgelegt. In der Anodenkammer werden 1 307 g einer 11,6 gew.-%igen methanolischen Natriumacetatlösung vorgelegt. Die Elektrodialyse wird bei 23 °C durchgeführt und nach 24 Stunden abgebrochen.

Es entstehen 66 g freier Acetessigsäuremethylester. Im Katholytkreislauf entstehen 48 g und im Konzentratkreislauf 47 g Natriummethylat.

## Patentansprüche

1. Verfahren zur Herstellung von Ketoverbindungen aus den zugehörigen Alkalisalzen,
dadurch gekennzeichnet,
daß man die Alkalisalze in einem nichtwäßrigen Medium ohne Zufuhr von systemfremder Säure elektrodialytisch protoniert und dabei die Ketoverbindungen freisetzt.

2. Verfahren nach Anspruch 1,
dadurch gekennzeichnet,
daß man die Protonierung in einer elektrodialytischen Zelle mit 2n + 2 Kammern, wobei n eine Zahl von 1 bis 50 ist, durchführt.

3. Verfahren nach Anspruch 2,
dadurch gekennzeichnet,
daß n eine Zahl von 1 bis 10 ist.

4. Verfahren nach Anspruch 1,
dadurch gekennzeichnet,
daß man β-Ketoester aus der Salzform herstellt.

5. Verfahren nach Anspruch 4,
dadurch gekennzeichnet,
daß man β-Ketolactone herstellt.

6. Verfahren nach Anspruch 1,
dadurch gekennzeichnet,
daß man als Nebenprodukt ein Alkalialkoholat herstellt.

7. Verfahren nach Anspruch 1,
dadurch gekennzeichnet,
daß man die Ketoverbindungen bei -20 bis +90 °C, vorzugsweise bei 10 bis 50 °C, gewinnt.

8. Verfahren nach Anspruch 4,
dadurch gekennzeichnet,
daß man dabei gleichzeitig elektrodialytisch eine Umesterung durchführt.

## Claims

1. A process for the preparation of a keto compound from a corresponding alkali metal salt, characterized in that the alkali metal salt is protonated by electrodialysis in a non-aqueous medium without the addition of acid which is foreign to the system, and the keto compound is thereby liberated.

2. A process according to claim 1, characterized in that the protonation is carried out in an electrodialysis cell having 2n + 2 chambers, where n is a number from 1 to 50.

3. A process according to claim 2, characterized in that n is a number from 1 to 10.

4. A process according to claim 1, characterized in that a β-keto ester is prepared from the salt form.

5. A process according to claim 4, characterized in that a β-keto lactone is prepared.

6. A process according to claim 1, characterized in that an alkali metal alcoholate is produced as by-product.

7. A process according to claim 1, characterized in that the keto compound is obtained at from -20 to +90°C, preferably at from 10 to 50°C.

8. A process according to claim 4, characterized in that a transesterification is carried out simultaneously with electrodialysis.

## Revendications

1. Procédé pour la fabrication de composés cétoniques à partir des sels alcalins correspondants,
caractérisé en ce que
les sels alcalins sont protonés par électrodialyse dans un milieu non aqueux sans addition d'acide externe au système et que l'on libère ainsi les composés cétoniques.

2. Procédé selon la revendication 1,
caractérisé en ce que
la protonation est réalisée dans une cellule d'électrodialyse comportant 2n + 2 chambres, où n est un nombre compris entre 1 et 50.

3. Procédé selon la revendication 2,
caractérisé en ce que
n est un nombre compris entre 1 et 10.

4. Procédé selon la revendication 1,
caractérisé en ce que
l'on fabrique des esters β-cétoniques à partir de leur forme saline.

5. Procédé selon la revendication 4,
caractérisé en ce que
l'on fabrique des lactones β-cétoniques.

6. Procédé selon la revendication 1,
caractérisé en ce que
l'on fabrique comme produits secondaires des alcoolates alcalins.

7. Procédé selon la revendication 1,
caractérisé en ce que
l'on obtient les composés cétoniques à des températures comprises entre -20 et + 90°C, de préférence entre 10 et 50°C.

8. Procédé selon la revendication 4,
caractérisé en ce que
l'on réalise par électrodialyse simultanément une réestérification.
